# EUROPEAN PATENT APPLICATION

(11) **EP 1 530 042 A1**
(43) Date of publication of application: **11.05.2005**
(21) Application number: 04254553.3
(22) Date of filing: 29.07.2004
(51) Int. Cl.: G01N 27/416, G01N 33/00

(54) **Self-testing gas detector**

(30) Priority: 10.11.2003 GB 0326188
(71) Applicant: Kidde IP Holdings Limited, Slough, Berkshire SL3 0HB (GB)
(72) Inventor: Harvey, Ann Marie, Pangbourne, Berkshire RG8 7JS (GB)
(74) Representative: Foster, Mark Charles

(57) **Abstract**

A method of calibrating a combined carbon monoxide and carbon dioxide detector, and a combined carbon monoxide and carbon dioxide detector comprising a carbon monoxide sensor, a carbon dioxide sensor, and a carbon monoxide and carbon dioxide gas generator are disclosed. The method of self-testing the carbon monoxide and carbon dioxide detector comprises the steps of controlling the carbon monoxide and carbon dioxide gas generator to generate a known quantity of carbon monoxide and carbon dioxide; monitoring the response of the carbon monoxide sensor to the known quantity of carbon monoxide; monitoring the response of the carbon dioxide sensor to the known quantity of carbon dioxide; and correcting the calibration of the carbon monoxide sensor and/or the carbon dioxide sensor when the response is outside of a predetermined range.

## Description

This invention relates to a combined carbon monoxide and carbon dioxide detector for detecting fires, with an integrated self-testing facility, and a method of self-testing for a carbon monoxide and carbon dioxide detector.

According to one aspect of the invention, there is provided a gas detector including a carbon monoxide sensor, a carbon dioxide sensor, a carbon monoxide generator, a carbon dioxide generator, means for selectively activating the carbon monoxide generator and the carbon dioxide generator, and means for analysing the response to the generated carbon monoxide and carbon dioxide by the carbon monoxide sensor and the carbon dioxide sensor.

According to a second aspect of the invention, there is provided a combined carbon monoxide and carbon dioxide self-testing detector, comprising a carbon monoxide sensor; a carbon dioxide sensor; and combined carbon monoxide and carbon dioxide gas generator.

According to a third aspect of the invention, there is provided a method of calibrating a carbon monoxide and carbon dioxide detector having a carbon monoxide sensor, a carbon dioxide sensor and a carbon monoxide and carbon dioxide generator in gaseous communication, including the steps of controlling the carbon monoxide and carbon dioxide gas generator to produce a known quantity of carbon monoxide and carbon dioxide; monitoring the response of the carbon monoxide sensor to the known quantity of carbon monoxide; monitoring the response of the carbon dioxide sensor to the known quantity of carbon dioxide; and correcting the calibration of the carbon monoxide sensor and/or the carbon dioxide sensor when the response is outside of a predetermined range.

The use of carbon monoxide or carbon dioxide detectors to detect fires is known, and all fires involving carbonaceous fuels produce varying amounts of carbon monoxide and carbon dioxide. The idea of using a carbon monoxide detector to detect slow, smouldering fires is gaining acceptance within the fire detection industry. It offers the potential to be used in areas that would cause conventional ionisation smoke detectors to produce a false alarm, such as laundry rooms. Carbon dioxide detectors are known to be particularly suited to sensing flaming fires.

Combined carbon monoxide and carbon dioxide fire detectors are therefore able to detect both slow smouldering and flaming fires without generation of false alarms due to water vapour or dust. Using a "rate of rise" measurement, as opposed to simply monitoring the level of gas, makes the detector less prone to false alarms from natural fluctuations in ambient levels of carbon monoxide or carbon dioxide:

It is advantageous to be able to test such detectors to check that they are functioning correctly. One method for testing is a "self-calibration" approach, whereby the reaction of the detector to small amounts of gas produced by a generator is monitored, and this method is particularly useful as it removes the need for human intervention. It is normal practice to use hydrogen as a calibration gas for carbon monoxide sensors. However the behaviour of the detector to hydrogen is not the same as its behaviour towards carbon monoxide.

US patent No. 6,365,022 (Alphasense Ltd) relates to a potentiometric carbon dioxide sensor operable at room temperature. There is, however, no "self-test" facility associated with this carbon dioxide sensor.

US patent application publication No. 2003/0145644 (Kidde Portable Equipment, Inc.) describes a self-calibrating carbon monoxide detector comprising a carbon monoxide sensor, a controller and a carbon monoxide gas generator. The function of the detector is primarily to sense carbon monoxide levels in domestic situations, and the controller can command the gas generator to produce carbon monoxide and then monitor the electrical output of the carbon monoxide sensor to ensure its proper operation. A membrane electrode assembly, wherein an ion exchange membrane is used as the electrolyte and the electrodes are made of carbon, electrolyses water held within the electrolyte, with hydrogen ions migrating to the negative electrode and oxygen ions migrating to the positive electrode. The hydrogen ions at the negative electrode react with gaseous oxygen to produce water. Hydrogen evolution is therefore suppressed, and this holds distinct advantages over similar approaches which evolve hydrogen as the test gas, for reasons outlined hereinbefore. Conversely, the oxygen ions at the positive electrode react with the surface of the positive electrode to produce carbon monoxide and carbon dioxide, as required to self-test the detector.

For a better understanding of the present invention, a combined carbon monoxide and carbon dioxide self-testing detector will now be described by way of example, with reference to the accompanying drawings in which:-
Figure 1 shows a general arrangement for a combined carbon monoxide and carbon dioxide self-testing detector;
Figure 2 shows the upper part of the cell layer and the filter material container of Figure 1 in greater detail;
Figure 3 shows the lower part of the cell layer, the water reservoir and the electrode contact pins of Figure 1 in greater detail;
Figure 4 shows a plot of the carbon dioxide produced by a generator contained in the detector against time;
Figure 5 shows a plot of the carbon monoxide produced by a generator contained in the detector against time; and
Figure 6 shows a plot of the concentration of carbon monoxide, carbon dioxide and oxygen against time recorded for a Class A type test fire inside an 8m³ test chamber.

In the drawings, like elements are generally designated with the same reference numeral.

The detector described in US publication 2003/0145644 evolves carbon monoxide and carbon dioxide typically in the ratio 95% carbon dioxide to 5% carbon monoxide. The detector is, however, unable to detect carbon dioxide, and so self-testing of the carbon monoxide sensor only is performed.

The embodiment of the present invention to be described utilises the evolved carbon dioxide produced by the gas generator of a self-testing carbon monoxide detector (for example, that of US publication 2003/0145644), in addition to a room temperature carbon dioxide sensor to create a combined carbon monoxide and carbon dioxide self-testing detector for sensing both slow smouldering and flaming fires without generation of (or at least a reduction in the generation of) false alarms due to particulates such as water vapour or dust.

In order to test the correct operation of the combined carbon monoxide and carbon dioxide detector, an electrical pulse is applied to the gas generator in order to produce a known amount of carbon monoxide and carbon dioxide, and the effect of this on the output of the sensor is observed. The amount of gas produced by electrolysis is directly proportional to the number of electrons that pass through the generator. By controlling both the current and duration of the electrical pulse used to electrolyse the water, it is therefore possible to control the volume of gas produced with each electrical pulse. The controller contains a constant current source and a timing process control element, and so the controller is able to control the generator to produce a known quantity of carbon monoxide and carbon dioxide. The behaviour of the particular sensor in response to the amount of carbon monoxide and carbon dioxide produced as a result of the current applied to the gas generator should produce a characteristic response in the sensor.

A single detector is created with the ability to detect both carbon monoxide and carbon dioxide individually, as well as being able to self-test by creating known amounts of carbon monoxide and carbon dioxide and measuring the response of the carbon monoxide and carbon dioxide sensors to this amount of gas. The operation of both the carbon monoxide sensor and the carbon dioxide sensor is possibly affected by the water content of the electrolyte contained within the sensor and, as described hereinbefore, the generation of carbon monoxide and carbon dioxide is brought about by the electrolysis of water and subsequent electrode reactions. Neither of the sensors nor the gas generator consume water during their operation so it is possible to use the same water reservoir for all three components. The detector could be no more than 50 mm in diameter, so that it would fit into a range of typical known detector housings.

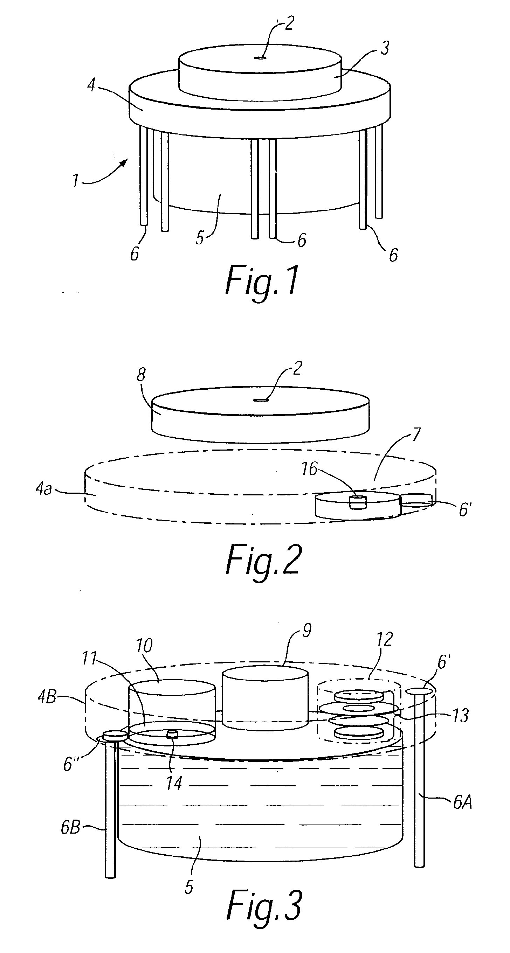

Figure 1 shows a general arrangement for a combined carbon monoxide and carbon dioxide self-testing detector 1. This detector 1 comprises a gas inlet 2, a filter material container 3, a cell layer 4, a water reservoir 5 and three pairs of electrode contact pins 6, one pair of electrode contact pins 6 being required for each cell contained within the cell layer 4. The outer casing of the detector 1 could be made from any suitable plastic (it should preferably not be styrene based). The electrical contacts 6 are moulded into the casing of the detector 1 and allow contact to respective electrodes (to be described below). The volume of the filter material in the region 3 should be minimised to reduce the dilution of the gas produced by the generator cell. The filter material is positioned between the diffusion limiting gas access hole 2 and the cell layer 4, as this reduces the concentration of contaminants that the filter is exposed to during its lifetime. Hence the volume of the filter material could be reduced without affecting its ability to protect the internal cell components from contamination. The cell layer 4 includes respective cells containing carbon monoxide sensor components, carbon dioxide sensor components and carbon monoxide and carbon dioxide gas generator components.

Figure 2 shows an upper part of the cell layer 4A and the filter material container 3 of Figure 1 in greater detail. In this drawing, a stainless steel disc 7 of one of the cells containing either the carbon monoxide sensor components, the carbon dioxide sensor components or the carbon monoxide and carbon dioxide gas generator components is shown in the cell layer 4. The disc 7 is in electrical contact with a head portion 6' of contact pin 6. The disc 7 is moulded into the casing of the detector 1, and includes a non-diffusion limiting gas access hole 16. Although only one disc 7 is shown in Figure 2, for the sake of clarity, it should be understood that a disc 7 of generally the same configuration will be provided for each of carbon monoxide sensing, carbon dioxide sensing and carbon monoxide and carbon dioxide gas generation entities.

Figure 3 shows a lower part of the cell layer 4B, the water reservoir 5 and the electrode contact pins 6 of Figure 1 in greater detail. Three generally cylindrical wells 9,10 and 12 are formed in the lower part of the cell layer 4B. Well 9 includes the carbon monoxide sensor elements, well 10 includes the carbon dioxide sensor elements, and well 12 includes the carbon monoxide and carbon dioxide gas generator elements. Each of these wells 9,10 and 12 is provided with a respective stainless steel disc 7 of the type shown in Figure 2. Each of the discs 7 is positioned with respect to its associated well 9,10 and 12 so as to form an end cap to that cell, whilst allowing the passage of gas to/from the well 9,10 and 12 through the gas access hole 16. Each of the discs 7 is an electrical contact with the head portion 6' of a respective contact pin 6 in the manner shown in Figure 2.

At the base of each of the wells 9,10 and 12, a further disc 11 is provided with a hole 14 provided therein for allowing water vapour transport therethrough.

Each of the discs 11 is positioned in electrical contact with a head portion 6" of the contact pin 6B associated with that cell.

Each cell has associated with it two contact pins 6. A first contact pin 6A is provided in electrical contact with a working electrode of the cell (through adjacent disc 7) and a second contact pin 6B is provided in electrical contact with a detection electrode of each cell (through adjacent disc 11).

The well 12 is shown to contain cell components 13. It should be appreciated that each of the wells 9,10 and 12 will include a set of cell components provided to perform the specified function of that cell. The cell components provided for each cell will be described in more detail herein below.

Carbon monoxide is sensed by a cell 9 formed in the cell layer 4 acting as a carbon monoxide sensor. In this sensor, the cell components 13 comprise the working electrode, the reference electrode and a solid electrolyte. The electrodes and may have the structure described in US patent No. 6,200,443 (Atwood Industries Inc.). Each electrode preferably comprises a mixed ionic-electronic conductive material and the solid electrolyte preferably comprises a membrane of a perfluorosulfonate ionomer. Introduction of carbon monoxide to the working electrode produces an oxidation reaction at the working electrode whereby carbon monoxide is converted into carbon dioxide and protons and electrons are liberated. The protons migrate across the solid electrolyte to the reference electrode, where they subsequently react with electrons and oxygen to form water in a reduction reaction. This electrochemical reaction generates an electrical signal which is proportional to the concentration of carbon monoxide. In such solid ion conducting electrolyte membranes, the rate of ion migration is affected by the water content of the electrolyte. The water reservoir 5 therefore allows the slow evaporation of water through the hole 14 provided in the disc 11, supplying enough water vapour to keep the sensor at a constant hydration level for many years.

Carbon dioxide is sensed by the cell 10 formed in the cell layer 4 acting as a carbon dioxide sensor. In this sensor, the cell components 13 comprise the working electrode, the reference electrode and a solid electrolyte. These components may be of the type described in U.S. Patent No. 6,365,022 (Alphasense Ltd). The reference electrode comprises silver. The working electrode preferably comprises a thin platinum black layer permeable to carbon dioxide. The solid electrolyte comprises a silver carbonate, silver rubidium iodide and PTFE binder layer, and a silver rubidium iodide and PTFE powder layer, capable of transmitting silver ions. Upon the introduction of carbon dioxide, a flux of silver ions is moved to the reference electrode, corresponding to the partial pressure of the gaseous carbon dioxide in the gas to be detected. This gives rise to an ion gradient between the electrodes, and the concentration of carbon dioxide can be deduced by measuring the resultant electrical signal.

Carbon monoxide and carbon dioxide are generated by the cell 12 formed in the cell layer 4 acting as a gas generator. In this assembly, both the working and reference electrodes comprise carbon, between which an ion exchange membrane is sandwiched. Preferably, the electrodes comprise carbon black and ion exchange polymer and no platinum. Water held within the ion exchange membrane and provided by the water reservoir 5 is electrolysed. Oxygen ions subsequently migrate to the positive electrode, and react with the surface of the positive electrode to produce carbon monoxide and carbon dioxide. In order to facilitate electrolysis in a controlled manner, an electrical pulse of known current and duration is applied to the gas generator means in order to produce a known amount of gas.

The carbon monoxide sensor, the carbon dioxide sensor, and the carbon monoxide and carbon dioxide generator are each provided with water vapour by the common water reservoir 5. The water reservoir 5 provides for the carbon monoxide sensor sufficient water vapour to keep the sensor at a constant hydration level over an extended period of time. The water reservoir 5 provides for the carbon dioxide sensor an environment with relatively constant humidity in order to extend the range over which carbon dioxide can be accurately sensed. The water reservoir 5 provides for the carbon monoxide and carbon dioxide generator with water required by the ion exchange membrane. Providing the two sensors and the generator with their common reservoir allows the overall size of the detector 1 to be reduced. Neither the two sensors nor the gas generator consume water during their operation. However, some water vapour will inevitably be released from the casing. The size of the reservoir is selected so that the detector will have an acceptable lifetime before all the water in the reservoir escapes. Because the detector of the present embodiment will generally release no more water vapour than that of a prior art device performing only one of the carbon monoxide detection, carbon dioxide detection, carbon monoxide generation and carbon dioxide generation functions, the volume of water, and thus the size of the reservoir, required by the present embodiment is the same as any one of those single-function prior art devices. The water reservoir of the present embodiment will supply the required water vapour over an extended period of time - providing the detector 1 with a long working life.

Components of the carbon monoxide sensor, the carbon dioxide sensor, and the combined carbon monoxide and carbon monoxide generator, may be separated by plastic discs or polywashers which have a central hole. These provide electrical insulation and mechanical separation of components where required and also control the passage of gases. Such discs or polywashers are not, however, considered necessary for the combined carbon monoxide and carbon dioxide generator.

In the combined carbon monoxide and carbon dioxide gas generator where there are no unwanted side reactions competing with the desired electrochemical reaction, the amount of carbon monoxide and carbon dioxide gases produced is directly proportional to the number of electrons that pass through the generator. The number of electrons is determined by the time integral of the current that passes through the generator. Testing and self-calibration of the detector 1 can be performed by applying an electrical pulse to the pins 6A,6B of the generator cell 12 and then observing the effect on the output of the carbon monoxide and carbon dioxide sensors by measuring the current at the respective pin pairs 6A,6B of each of the sensors. Controlling the volume of gas produced with each electrical pulse can best be achieved by controlling both the pulse current and the duration of the pulse applied to the pins 6A,6B of the generator cell 12. The drive circuit for the gas generator preferably includes a constant current source. Such a current source delivers a preset current irrespective of the voltage required. To control the current pulse duration, a timing process control element is included with the current source.

Typically, the generator requires less than 10mA of current to be applied for a few seconds.

The production of carbon monoxide and carbon dioxide by the generator cell 12 may be temperature dependent. To take into account the temperature dependence, the magnitude of the applied current may be varied as a function of the ambient temperature, or the duration of the applied pulse could be varied as a function of the ambient temperature. Whichever factor is varied, the variation is based on ambient temperature so that the amount of carbon monoxide produced by the generator is a known quantity.

Although the production of carbon monoxide and carbon dioxide in the generator cell 12 may be temperature dependent, the ambient temperature has little or no effect on the carbon monoxide and carbon dioxide sensors over the range of temperature where a domestic detector 1 might be expected to operate. Therefore, temperature compensation of the sensor response may not be required.

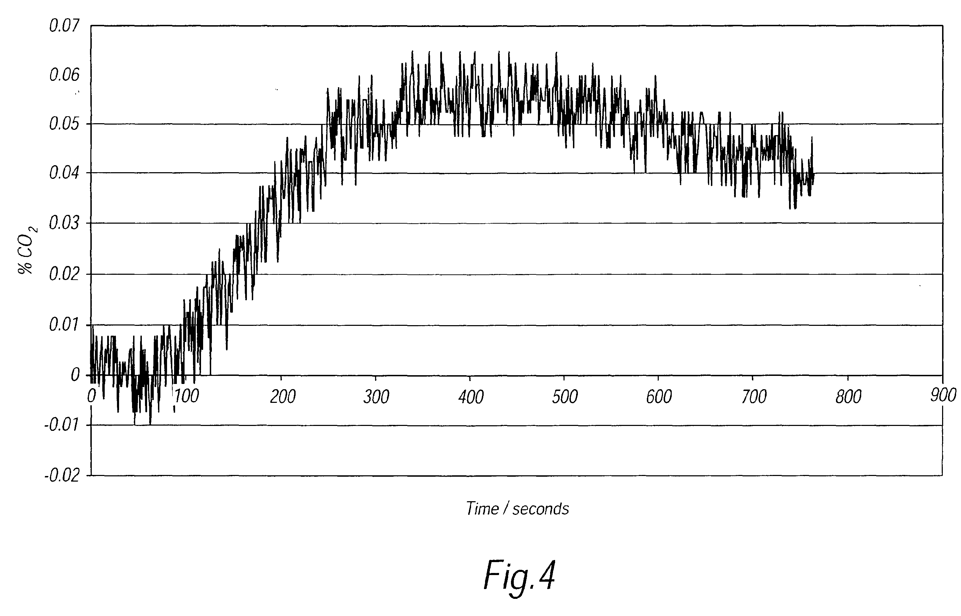

Figure 4 shows a plot of the carbon dioxide produced by the generator cell 12 operated for 40 seconds at 8mA showing percent carbon dioxide against time.

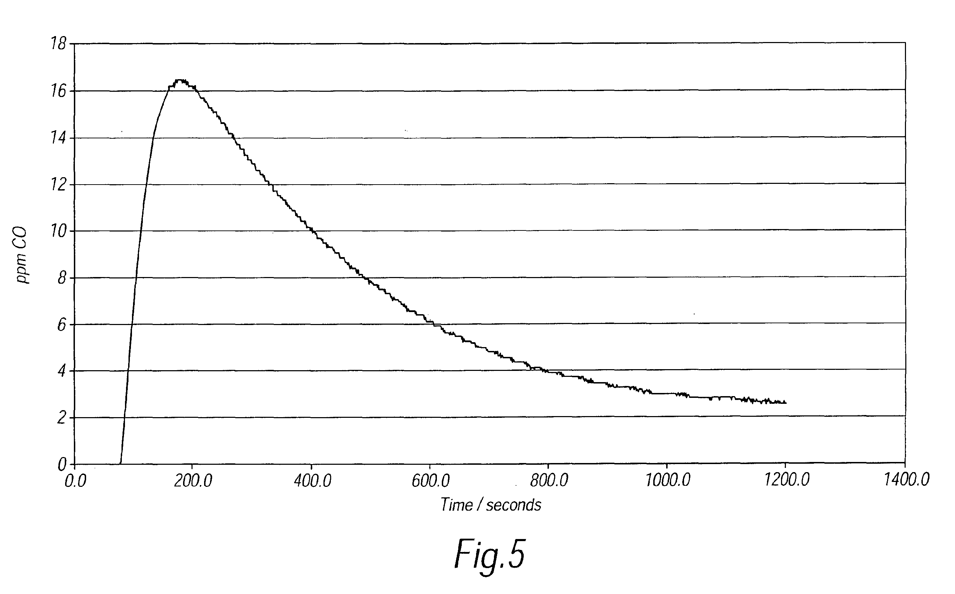

Figure 5 shows a plot of the carbon monoxide produced by the generator cell 12 operated for 20 seconds at 8mA showing parts per million CO against time.

The quantity of carbon monoxide and carbon dioxide produced by the generator cell 12 expected in response to a given electrical impulse is therefore known. By monitoring the electrical responses of the carbon monoxide sensor cell 9 and the carbon dioxide sensor cell 10, the actual responses of the sensor cells 9 and 10 can be compared with the expected responses produced when the generator cell 12 is energised. If the measured response differs from the expected response a repeat test, for example, could be performed. The repeat test would be performed one or more times. If the divergence between the measured response and the expected response differed consistently, the detector 1 can be re-calibrated. This will be discussed further below.

In normal operation of the detector 1, the generator cell 12 will not be activated. The levels of ambient carbon monoxide and carbon dioxide will be measured continuously by the sensor cells 9 and 10. The electrical currents produced by the respective pins 6A and 6B of each of the cells will be detected by control circuitry or a control algorithm (not shown) provided in association with the sensor (this could be provided with the detector 1 as a unit or could be situated remotely from the detector 1). The current produced at the pins 6A,6B of the carbon monoxide sensor 9 and the carbon dioxide sensor 10 for a given amount of carbon monoxide/carbon dioxide will be known. The control electronics/algorithm will accordingly be able to associate a value of carbon monoxide and carbon dioxide in the ambient air with a particular current measurement from the carbon monoxide sensor cell 9 and the carbon dioxide sensor cell 10. These values of carbon monoxide and carbon dioxide can then be used in any desired manner. For example, the values may be displayed, or an audible or visual representation could be output by the detector 1 indicating when these values pass a particular threshold.

The detector 1 may be re-calibrated or tested by energising the generator cell 12 in the manner described above. If, as described above, the current or voltage generated by the carbon monoxide sensor cell 9 or the carbon dioxide sensor cell 10 differ from the expected results, the control electronics or algorithm can adjust the value of carbon monoxide/carbon dioxide that it associates with a given current or voltage from the carbon monoxide sensor cell 9/carbon dioxide sensor cell 10. In this way, the accuracy of the sensor cells 9 and 10 can be maintained. The self testing/re-calibration may be performed automatically periodically (being initiated by the control electronics/algorithm), or may be activated by user intervention (for example by the user actuating the switch). The carbon monoxide and carbon dioxide sensors can be re-calibrated separately from each other.

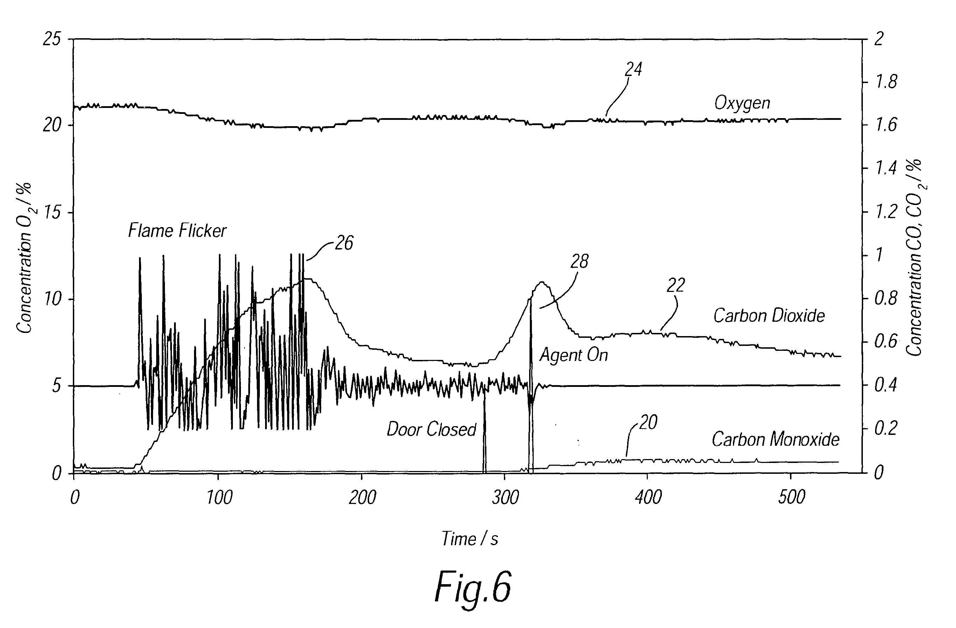

Figure 6 shows a plot of the concentration of carbon monoxide 20, carbon dioxide 22 and oxygen against time recorded for a Class A type test fire inside an 8m³ test chamber. Flame flicker 26 measured by an infra-red detector is also shown in Figure 6. A wooden crib was ignited using a heptane fire that lasted for approximately 2.5 minutes. It is the heptane fire that produces the initial rapid rise in carbon dioxide levels and flame flicker. There is no significant deviation of oxygen levels. Initially, no carbon monoxide is produced. The levels of carbon dioxide detected inside the test vessel only reached approximately 1% v/v during the heptane fire stage. It is possible for the ambient carbon dioxide levels in a closed, occupied room to reach this level over many hours, making a system based on a peak reading unreliable and prone to false alarm. However, because a rapid rise in the carbon dioxide level was observed during the fire test, it is considered that a rate of rise measurement of carbon dioxide would provide a more reliable indication of the presence of a fire without producing unnecessary false alarms. Carbon monoxide levels only begin to rise after the wood crib fire has been extinguished by application of a fire suppressant at 26. This is thought to be caused by continued smouldering, indicating the value of a carbon monoxide sensor for smouldering fire detection.

The control electronics/algorithm of the detector 1 may be programmed with a model of the variation of carbon dioxide and carbon monoxide in response to a flaming fire and a smouldering fire. The variation of the quantity of carbon monoxide and carbon dioxide detected can be compared to this model to allow the detector 1 to use artificial intelligence to determine whether a flaming fire or a smouldering fire is likely to be present.

The detector 1 is therefore able to detect both slow smouldering and flaming fires without generation of false alarms due to water vapour or dust. Using a rate of rise measurement will reduce the occurrence of false alarms (or eliminate false alarms) due to natural ambient fluctuations in background levels of carbon dioxide. However, the background level of carbon dioxide may also be monitored by the detector and this could be used as part of an automated home control system to activate ventilation systems when ambient carbon dioxide levels are too high.

In an alternative embodiment of the invention, the water reservoir 5 is not provided, and there is no water supply made available to the carbon monoxide detector, the carbon dioxide detector, and the combined carbon monoxide and carbon dioxide generator. The carbon dioxide sensor described in US Patent No. 6365022 does not employ a water reservoir. International patent publication No. WO 02/11225 (City Technology) discloses a carbon monoxide sensor using an alternative polymeric electroyte system that does not include a perfluorosulfonated ionomer. Such a material may also be used to form a carbon monoxide and carbon dioxide generator, in addition to a carbon monoxide sensor.

The publications referred to herein are fully incorporated herein by reference.

It will be appreciated that other carbon monoxide and carbon dioxide sensor configurations could be used to create a combined carbon monoxide and carbon dioxide self-testing detector. The examples given herein are not intended to, and should not be taken to, limit the scope of the invention. For example, the carbon dioxide sensor could be an infra-red or a semiconductor sensor.

## Claims

1. A gas detector including a carbon monoxide sensor (9), a carbon dioxide sensor (10), a carbon monoxide generator (12), a carbon dioxide generator (12), means (6) for selectively activating the carbon monoxide generator and the carbon dioxide generator, and means (6) for analysing the response to the generated carbon monoxide and carbon dioxide by the carbon monoxide sensor and the carbon dioxide sensor.

2. The detector of claim 1, wherein each of the carbon monoxide sensor (9), the carbon dioxide sensor (10), the carbon monoxide generator (12) and the carbon dioxide generator (12) are provided in fluid communication with a water supply (5).

3. The detector of claim 2, wherein the water supply (5) is provided by a water reservoir that is in fluid communication with each of the carbon monoxide sensor (9), carbon dioxide sensor (10), the carbon monoxide generator (12) and the carbon dioxide generator (12).

4. A combined carbon monoxide and carbon dioxide self-testing detector, comprising:
a carbon monoxide sensor (9);
a carbon dioxide sensor (10); and
combined carbon monoxide and carbon dioxide gas generator (12).

5. The detector of any one of claims 1 to 4, including means for determining the rate of rise in the levels of sensed carbon monoxide and/or carbon dioxide.

6. The detector of any one of claims 1 to 5, wherein the carbon monoxide sensor (9) is an electrochemical sensor.

7. The detector of any one of claims 1 to 6, wherein the carbon dioxide sensor (10) is an electrochemical sensor.

8. The detector of any one of claims 1 to 7, wherein the carbon monoxide and/or carbon dioxide gas generator (12) comprises an ion exchange membrane having electrodes deposited on the upper surface and lower surface thereof.

9. The detector of claim 8, wherein the said electrodes contain carbon.

10. The detector of claim 6, wherein the carbon monoxide sensor (9) contains a first electrode, a second electrode and a solid electrolyte.

11. The detector of claim 10, wherein the first and second electrodes comprise a mixed ionic-electronic conductive material.

12. The detector of claim 11, wherein the solid electrolyte comprises a membrane of a perfluorosulfonate ionomer.

13. The detector of claim 7, wherein the carbon dioxide sensor (10) contains an electrolytic cell comprising a reference electrode, a detection electrode and a solid electrolyte.

14. The detector of claim 13, wherein the solid electrolyte comprises an electrocatalyst and silver carbonate.

15. The detector of claim 13 or 14, wherein the detection electrode comprises a platinum black layer permeable to carbon dioxide.

16. The detector of claim 13, 14 or 15, wherein the solid electrolyte is capable of transmitting silver ions.

17. The detector of claim 13,14,15 or 16, wherein the solid electrolyte comprises a silver rubidium iodide.

18. The detector of any one of claims 13 to 17, wherein the reference electrode has the same characteristics as the detection electrode.

19. A method of calibrating a carbon monoxide and carbon dioxide detector having a carbon monoxide sensor (9), a carbon dioxide sensor (10) and a carbon monoxide and carbon dioxide generator (12) in gaseous communication, including the steps of:
controlling the carbon monoxide and carbon dioxide gas generator (12) to produce a known quantity of carbon monoxide and carbon dioxide;
monitoring the response of the carbon monoxide sensor (9) to the known quantity of carbon monoxide;
monitoring the response of the carbon dioxide sensor (10) to the known quantity of carbon dioxide; and
correcting the calibration of the carbon monoxide sensor (9) and/or the carbon dioxide sensor (10) when the response is outside of a predetermined range.

20. A method according to claim 19, wherein the generation of carbon monoxide and carbon dioxide is brought about by the electrolysis of water.

21. A method according to claim 20, wherein the water is electrolysed using an ion exchange membrane as the electrolyte.

22. A method according to claim 20 or claim 21, wherein the controller generates a known quantity of carbon monoxide and/or carbon dioxide by controlling the both the current and the duration of an electrical pulse used to electrolyse the water.

23. A method according to claim 19,20,21 or 22, wherein the peak sensor signal of the carbon monoxide sensor and/or the carbon dioxide sensor in response to a known quantity of carbon monoxide and/or carbon dioxide is monitored.

24. A method according to claim 19,20,21 or 22, wherein the integrated value of the sensor signal between two given times of the carbon monoxide sensor and/or the carbon dioxide sensor in response to a known quantity of carbon monoxide and/or carbon dioxide is monitored.
